# EUROPÄISCHE PATENTSCHRIFT

(11) **EP 1 951 346 B1**
(45) Veröffentlichungstag und Bekanntmachung des Hinweises auf die Patenterteilung: **04.12.2013**
(21) Anmeldenummer: 06762060.9
(22) Anmeldetag: 16.06.2006
(51) Int. Cl.: A61M 16/00

(54) **SYSTEM ZUR FREISCHALTUNG VON BEDIENUNGSMODI AN EINEM MEHRTEILIGEN MEDIZINTECHNISCHEN GERÄT**
SYSTEM FOR ENABLING OPERATING MODES ON A MULTI-PART MEDICAL APPLIANCE
SYSTEME POUR ACTIVER DES MODES DE FONCTIONNEMENT D'UN APPAREIL MEDICAL TECHNIQUE CONSTITUE DE PLUSIEURS PIECES

(30) Priorität: 26.11.2005 DE 102005056477
(43) Veröffentlichungstag der Anmeldung: 06.08.2008
(73) Patentinhaber: Dräger Medical GmbH, 23558 Lübeck (DE)
(72) Erfinder: SCHERMEIER, Olaf, 23560 Lübeck (DE); KLAFKE, Marita, 23564 Lübeck (DE); OTTO, Andreas, 22941 Bargteheide (DE); WOTHA, Gerd, 23626 Warnsdorf (DE)
(74) Vertreter: UEXKÜLL & STOLBERG
(86) Internationale Anmeldenummer: PCT/EP2006/005779
(87) Internationale Veröffentlichungsnummer: WO 2007/059810

(56) Entgegenhaltungen:
- EP-A- 1 516 641
- DE-A1- 10 116 650
- US-A1- 2004 171 982
- US-A1- 2004 210 151

## Beschreibung

Die Erfindung betrifft ein System zur Freischaltung von Bedienungsmodi an einem mehrteiligen medizintechnischen Gerät. Bei den Bedienungsmodi handelt es sich um Funktionen, die in der Software eines medizintechnischen Gerätes, wie zum Beispiel eines Anästhesie- oder Beatmungsgerätes, bereits implementiert sind oder zu einem zukünftigen Zeitpunkt implementiert werden sollen. Ein Bedienungsmodus entspricht beispielsweise einem bestimmten maschinellen Beatmungsmodus, wie NIV®; AutoFlow®, SmartCare®. Ein Benutzer muss sich üblicherweise bei der Anschaffung entsprechender medizintechnischer Geräte festlegen, welche Bedienungs-, sprich Beatmungsmodi ihm zur Verfügung stehen sollen. Ändert sich das Anforderungsprofil an das medizintechnische Gerät, werden also zu einem späteren Zeitpunkt zusätzliche Beatmungsmodi gewünscht oder erweisen sich bereit vorhandene Beatmungsmodi als nicht mehr erforderlich, so muss bisher am Gerät selbst die entsprechende Softwaremodifikation vorgenommen werden. Die Erbringung dieser Serviceleistung vor Ort ist mit zusätzlichem Aufwand und Kosten verbunden.

Aus der DE 101 16 650 B4 ist es bekannt, über ein externes, separates Speichermedium in Form einer Chipkarte Bedienungsmodi freizuschalten oder zu sperren.

Die Aufgabe der Erfindung besteht in der Bereitstellung eines in Bezug auf die Handhabbarkeit im praktischen Einsatz verbesserten Systems zur Freischaltung von Bedienungsmodi an einem mehrteiligen medizintechnischen Gerät, welche insbesondere einem individuellen Patienten zugeordnet sind.

Die Lösung der Aufgabe erhält man mit den Merkmalen von Anspruch 1. Die Unteransprüche geben vorteilhafte Aus- und Weiterbildungen des Systems nach Anspruch 1 an.

Ein mehrteiliges medizintechnisches Gerät besteht aus dem Grundgerät und einem oder mehreren Zusatzgeräten oder Zubehörteilen, die für den Betrieb des medizintechnischen Gerätes erforderlich sind. Zusatzgeräte für ein Grundgerät in Form eines Anästhesie- oder Beatmungsgerätes sind beispielsweise Beatmungsschläuche, Beatmungsmasken, Beatmungsanfeuchter, Filter, Messeinheiten, CO₂-Absorber, welche speziell patientenindividuelle Zusatzgeräte sind, die für einen bestimmten Patienten zum Einsatz gelangen und im Allgemeinen nach dem Gebrauch entsorgt werden.

Die Freischaltung der Bedienungsmodi erfolgt in der Weise, dass bei ordnungsgemäßem mechanischem Anschluss eines Zusatzgerätes an das Grundgerät der Datenspeicher des Zusatzgerätes mit den auslesbaren Daten in die Sende- und Empfangsreichweite der Leseeinheit des Grundgerätes für die drahtlose Auslesung der Daten aus dem Datenspeicher gelangt. Der Datenspeicher ist insbesondere ein Transponder, also eine Empfangs-Sendeeinheit, die nach dem Abfrage-Antwort-System arbeitet. Ein vom Transponder mittels einer Antenne empfangenes, codiertes Abfragesignal wird entschlüsselt und.nach der Kennung und gegebenenfalls anderen Informationen der abfragenden Leseeinheit ausgewertet. Daraufhin wird automatisch ein codiertes, selektiv für die abfragende Leseeinheit bestimmtes Antwortsignal mit den gewünschten Daten für freizuschaltende Bedienungsmodi und gegebenenfalls mit zusätzlichen patientenindividuellen Zusatzdaten drahtlos vom Transponder übertragen. Die Daten werden in der Leseeinheit des Grundgerätes ebenfalls automatisch entschlüsselt und ausgewertet.

Für die einzelnen freigeschalteten Bedienungsmodi im medizintechnischen Gerät kann jeweils ein Zeitpunkt oder eine Dauer der Verfügbarkeit festgelegt werden, beispielsweise in Form eines für jeden freigeschalteten Bedienungsmodus geführten ZeitKontos, von dem Zeiteinheiten abgebucht werden können.

Der Datenspeicher im Zusatzgerät kann in einer speziellen Ausführungsform auch beschreibbar und die komplementäre Leseeinheit im Grundgerät mit einer Schreibeinheit für den,Datenspeicher kombiniert sein.

Speziell ist das mechanisch an das Grundgerät anschließbare Zusatzgerät ein spezifisches Zubehörteil, welches zur einmaligen Verwendung bestimmt ist und vom Gerätebenutzer ohnehin für die bestimmungsgemäße Funktion des medizintechnischen Gerätes am Grundgerät angeschlossen wird.

Das Grundgerät, insbesondere ein Anästhesie- oder Beatmungsgerät, befindet sich vor dem mechanischen Anschluss des Zusatzgerätes, also beispielsweise eines Beatmungsschlauches, einer Beatmungsmaske, eines Filters oder eines Beatmungsanfeuchters, in seiner Grundkonfiguration, das heißt bestimmte optionale Bedienungsmodi sind noch nicht freigeschaltet. Wird nun das spezielle Zusatzgerät zur einmaligen, für einen individuellen Patienten bestimmten Verwendung angeschlossen, so wird der Betrieb der bestimmten, speziell einem individuellen Patienten zugeordneten Bedienungsmodi für die Dauer des Anschlusses am Grundgerät freigeschaltet. Wird das spezifische Zusatzgerät wieder vom Grundgerät entfernt, so stehen die optionalen Bedienungsmodi nicht mehr zur Verfügung. Wird das spezifische, patientenindividuelle Zusatzgerät an einem anderen Grundgerät angeschlossen, zum Beispiel, wenn sich der Patient an einem anderen Grundgerät befindet, so stehen die optionalen Bedienungsmodi gegebenenfalls hier zur Verfügung. Optional kann eine bestimmte Laufzeit oder Benutzungsdauer mit dem Zusatzgerät oder Zubehörteil verbunden sein, wonach die Freischaltung erlischt, zum Beispiel nach einer gewissen Anzahl von Betriebsstunden. Somit kann eine patientenindividuelle Konfiguration von medizintechnischen Geräten erfolgen. Zusätzlich können aufgrund der erfassten Daten die Gesamtkosten der Nutzung patientenindividuell erfasst werden, ohne dass das Handling der Geräte verändert wird.

Ein Ausführungsbeispiel wird mit Hilfe der einzigen, schematischen Figur erläutert, welche ein Anästhesie- oder Beatmungsgerät im Bereich einer gasführenden Schnittstelle zwischen dem Grundgerät 1 und einem Zusatzgerät 4 in Form eines Beatmungsschlauchs zeigt. Der Atemgasanschluss weist einen winkelveränderlichen männlichen Konnektor 10 auf. An diesem Konnektor 10 erfolgt der Anschluss des Beatmungsschlauches, indem eine dichtende Tülle 11 als weiblicher Konnektor mit dem männlichen Konnektor 10 verbunden wird.

Ein Transponder weist einen digitalen elektronischen Datenspeicher 2 mit einer Antenne 12 auf, welche speziell so in die Tülle 11 eingespritzt ist, dass ihre Windungen senkrecht zur Achse des Schlauchanschlusses gerichtet sind. Eine weitere Antenne 9 einer grundgeräteseitigen Leseeinheit 3, gegebenenfalls kombiniert mit einer Schreibeinheit, ist im Wesentlichen senkrecht zur Achse des fest mit dem Grundgerät 1 verbundenen Teiles des Atemgasanschlusses 13 angebracht. Auf diese Weise ergibt sich, dass bei ordnungsgemäß an das Grundgerät 1 angeschlossenem Zusatzgerät 4 in allen Positionen des winkelveränderlichen männlichen Konnektors 10 die Felder, die sich um die Antennen 9, 12 ausbilden, jeweils bezüglich der empfangenden Antenne eine Parallelkomponente aufweisen, so dass eine ausreichende induktive Kopplung für die drahtlose Übertragung von Signalen und Daten gegeben ist.

Der digitale elektronische Datenspeicher 2 enthält Informationen über die freizuschaltenden Bedienungsmodi, wie beispielsweise Beatmungsmodi, und Informationen, wie lange diese Modi mit dem entsprechenden Zusatzgerät freigeschaltet werden. Der Datenspeicher 2 ist durch Kleben, Schweißen oder Einspritzen mit der Tülle 11 verbunden. Nach Gebrauch wird der Beatmungsschlauch mitsamt Transponder entsorgt. Die Daten im Datenspeicher 2 sind so verschlüsselt, dass die Informationen nicht manipuliert werden können. Die Leseeinheit 3 im Grundgerät 1 liest die Daten über mindestens eine oder mehrere Antennen 9 ein, entschlüsselt sie und wertet sie aus. Je nach Auswertung werden die Bedienungsmodi, speziell Beatmungsmodi, im medizintechnischen Gerät freigeschaltet.

Wird ein herkömmliches Zubehörteil angeschlossen, werden die zusätzlichen Funktionen am medizintechnischen Gerät nicht bereitgestellt. Neben den genannten Daten können weitere patientenindividuelle oder zusatzgerätindividuelle Informationen im Datenspeicher 2 abgelegt sein.

Die Leseeinheit 3 ist mit der Elektronik des Grundgerätes 1 über eine Leitung 5 verbunden.

Speziell sind die Daten im Datenspeicher mittels eines Codes bei der Herstellung codiert und ist die Leseeinheit 3 mit einem symmetrischen Schlüssel für die Decodierung der Daten ausgestattet.

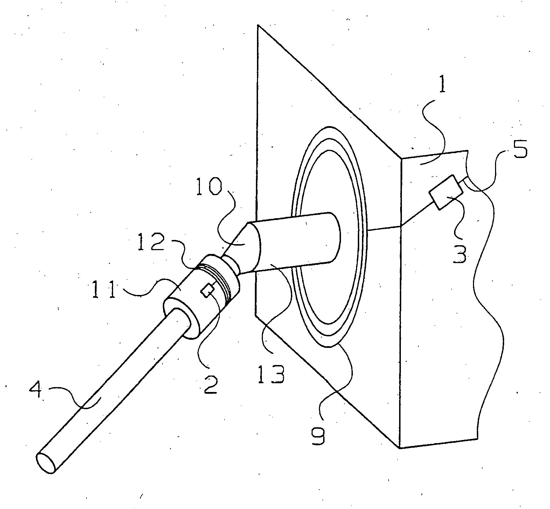

## Patentansprüche

1. System zur Freischaltung von Bedienungsmodi an einem mehrteiligen medizintechnischen Gerät mit einem Grundgerät (1) und mindestens einem anschließbaren Zusatzgerät (4) für das Grundgerät (1), wobei das Zusatzgerät (4) mit einem drahtlos auslesbaren Datenspeicher (2) und das Grundgerät (1) mit einer Leseeinheit (3) für den Datenspeicher (2) ausgestattet ist, wobei der Datenspeicher (2) Informationen über freizuschaltende Bedienungsmodi des Geräts enthält und wobei bei erfolgtem Anschluss des Zusatzgerätes (4) der Datenspeicher (2) in Sende-/Empfangsweite der Leseeinheit (3) für die Datenauslesung der Informationen aus dem Datenspeicher (2) gelangt, die Leseeinheit (3) die Informationen ausliest und die entsprechenden Bedienungsmodi freigeschaltet werden.

2. System nach Anspruch 1, **dadurch gekennzeichnet, dass** der Datenspeicher (2) des Zusatzgeräts (4) mit einem individuellen Patienten zugeordneten Daten versehen ist, welche zumindest einen Bedienungsmodus für den Patienten umfassen.

3. System nach Anspruch 1 oder 2, **dadurch gekennzeichnet, dass** der Datenspeicher (2) des Zusatzgeräts (4) mit für das Zusatzgerät (4) charakteristischen Daten versehen ist, so dass nur für das Zusatzgerät (4) charakteristische Bedienungsmodi im medizintechnischen Gerät freigeschaltet werden, insbesondere solche Bedienungsmodi, die einem individuellen Patienten zugeordnet sind.

4. System nach einem der vorhergehenden Ansprüche, **dadurch gekennzeichnet, dass** das Grundgerät (1) ein Anästhesie- oder Beatmungsgerät und das Zusatzgerät (4) ein Beatmungsschlauch, eine Beatmungsmaske, ein Filter, ein Beatmungsanfeuchter ist.

5. System nach einem der vorhergehenden Ansprüche, **dadurch gekennzeichnet, dass** der Datenspeicher (2) ein Transponder ist.

6. System nach Anspruch 4 oder 4 und 5, **dadurch gekennzeichnet, dass** die Bedienungsmodi Softwaremodule für Beatmungsverfahren eines Anästhesie- oder Beatmungsgerätes sind.

7. System nach einem der vorhergehenden Ansprüche, **dadurch gekennzeichnet, dass** die Leseeinheit (3) mit einer Schreibeinheit kombiniert und der Datenspeicher (2) beschreibbar ist.

8. System nach Anspruch 7, **dadurch gekennzeichnet, dass** der Datenspeicher (2) nach Freischaltung eines Bedienungsmodus am medizintechnischen Gerät mit für die Freischaltung charakteristischen Daten beschrieben ist.

9. System nach einem der vorhergehenden Ansprüche, **dadurch gekennzeichnet, dass** die Freischaltung von Bedienungsmodi für die Dauer des Anschlusses des Zusatzgerätes (4) erfolgt.

10. System nach einem der vorhergehenden Ansprüche, **dadurch gekennzeichnet, dass** die Freischaltung von Bedienungsmodi für einen im Datenspeicher (2) festgelegten Zeitpunkt und/oder eine festgelegte Dauer erfolgt.

11. System nach einem der vorhergehenden Ansprüche, **dadurch gekennzeichnet, dass** mittels des Datenspeichers (2) im medizintechnischen Gerät aktuell aktive Bedienungsmodi deaktivierbar sind.

12. System nach einem der vorhergehenden Ansprüche, **dadurch gekennzeichnet, dass** die Daten im Datenspeicher (2) des Zusatzgerätes (4) codiert sind und die Leseeinheit (3) mit einem komplementären, symmetrischen Schlüssel für die Decodierung der Daten ausgestattet ist.

13. System nach einem der vorhergehenden Ansprüche, **dadurch gekennzeichnet, dass** das Zusatzgerät (4) als Einmalartikel zur einmaligen Verwendung am Patienten ausgebildet ist.

## Claims

1. A system for enabling operating modes on a multipart medical device having a base device (1) and at least one connectable ancillary device (4) for the base device (1), wherein the ancillary device (4) is provided with a wirelessly readable data storage means (2) and the base device (1) is provided with a reading unit (3) for the data storage means (2), wherein the data storage means (2) contains information about operating modes of the device to be enabled and wherein upon established connection of the ancillary device (4) the data storage means (2) comes into transmission/receiving range of the reading unit (3) for the data reading of the information from the data storage means (2), the reading unit (3) reads the information and the corresponding operating modes are enabled.

2. The system according to claim 1, **characterized in that** the data storage means (2) of the ancillary device (4) is provided with data associated with an individual patient, which data comprise at least an operating mode for the patient.

3. The system according to claim 1 or 2, **characterized in that** the data storage means (2) of the ancillary device (4) is provided with data which are characteristic of the ancillary device (4), so that only operating modes characteristic of the ancillary device (4) are enabled in the medical device, in particular such operating modes which are associated with an individual patient.

4. The system according to any of the preceding claims, **characterized in that** the base device (1) is an anaesthetic or respiratory device and the ancillary device (4) is a respiratory hose, a respiratory mask, a filter, a respiratory humidifier.

5. The system according to any of the preceding claims, **characterized in that** the data storage means (2) is a transponder.

6. The system according to claim 4 or 4 and 5, **characterized in that** the operating modes are software modules for respiratory methods of an anaesthetic or respiratory device.

7. The system according to any of the preceding claims, **characterized in that** the reading unit (3) is combined with a writing unit and the data storage means (2) is writable.

8. The system according to claim 7, **characterized in that** subsequent to enabling an operating mode on the medical device the data storage means (2) has been written with data characteristic of the enabling.

9. The system according to any of the preceding claims, **characterized in that** the enabling of operating modes is effected for the duration of the connection of the ancillary device (4).

10. The system according to any of the preceding claims, **characterized in that** the enabling of operating modes is effected for a point in time defined in the data storage means (2) and/or a defined duration.

11. The system according to any of the preceding claims, **characterized in that** by means of the data storage means (2) operating modes currently active in the medical device can be deactivated.

12. The system according to any of the preceding claims, **characterized in that** the data in the data storage means (2) of the ancillary device (4) are encoded and the reading unit (3) is provided with a complementary, symmetrical key for the decoding of the data.

13. The system according to any of the preceding claims, **characterized in that** the ancillary device (4) is constructed as disposable article for single use on a patient.

## Revendications

1. Système pour activer des modes de fonctionnement d'un appareil médical technique constitué de plusieurs parties, comprenant un appareil de base (1) et au moins un appareil accessoire (4), pouvant être raccordé, pour l'appareil de base (1), dans lequel l'appareil accessoire (4) est équipé d'une mémoire de données (2) pouvant être soumise à une lecture sans fil, et l'appareil de base (1) est équipé d'une unité de lecture (3) pour la mémoire de données (2), la mémoire de données (2) contenant des informations sur des modes de fonctionnement à activer de l'appareil, et, une fois le raccordement de l'appareil accessoire (4) effectué, la mémoire de données (2) arrivant à portée d'émission/de réception de l'unité de lecture (3) pour la lecture de données des informations à partir de la mémoire de données (2), l'unité de lecture (3) lisant les informations, et les modes de fonctionnement correspondant étant activés.

2. Système selon la revendication 1, **caractérisé en ce que** la mémoire de données (2) de l'appareil accessoire (4) est pourvue de données qui sont associées à un patient individuel et englobent au moins un mode de fonctionnement pour le patient.

3. Système selon la revendication 1 ou 2, **caractérisé en ce que** la mémoire de données (2) de l'appareil accessoire (4) est dotée de données caractéristiques de l'appareil accessoire (4), de sorte que seuls des modes de fonctionnement caractéristiques de l'appareil accessoire (4) sont activés dans l'appareil médical technique, en particulier les modes de fonctionnement qui sont associés à un patient individuel.

4. Système selon l'une des revendications précédentes, **caractérisé en ce que** l'appareil de base (1) est un appareil d'anesthésie ou un respirateur artificiel, et l'appareil accessoire (4) est un tube de ventilation, un masque de ventilation, un filtre, un humidificateur respiratoire.

5. Système selon l'une des revendications précédentes, **caractérisé en ce que** la mémoire de données (2) est un transpondeur.

6. Système selon la revendication 4 ou 4 et 5, **caractérisé en ce que** les modes de fonctionnement sont des modules de logiciel pour des processus de ventilation d'un appareil d'anesthésie ou d'un respirateur artificiel.

7. Système selon l'une des revendications précédentes, **caractérisé en ce que** l'unité de lecture (3) peut être combinée avec une unité d'écriture et la mémoire de données (2) peut recevoir une écriture.

8. Système selon la revendication 7, **caractérisé en ce que**, après l'activation d'un mode de fonctionnement de l'appareil médical technique, la mémoire de données (2) est soumise à l'écriture de données caractéristiques de l'activation.

9. Système selon l'une des revendications précédentes, **caractérisé en ce que** l'activation de modes de fonctionnement est effectuée pour la durée de la connexion de l'appareil accessoire (4).

10. Système selon l'une des revendications précédentes, **caractérisé en ce que** l'activation de modes de fonctionnement est effectuée pour un instant défini dans la mémoire de données (2) et/ou pour une durée définie.

11. Système selon l'une des revendications précédentes, **caractérisé en ce que** la mémoire de données (2) permet de désactiver des modes de fonctionnement actuellement actifs dans l'appareil médical technique.

12. Système selon l'une des revendications précédentes, **caractérisé en ce que** les données dans la mémoire de données (2) de l'appareil accessoire (4) sont codées, et l'unité de lecture (3) est équipée d'un code symétrique complémentaire pour le décodage des données.

13. Système selon l'une des revendications précédentes, **caractérisé en ce que** l'appareil accessoire (4) est réalisé sous la forme d'un article jetable destiné à un usage unique sur le patient.
